# EUROPEAN PATENT SPECIFICATION

(11) **EP 3 099 664 B1**
(45) Date of publication and mention of the grant of the patent: **18.04.2018**
(21) Application number: 15702684.0
(22) Date of filing: 23.01.2015
(51) Int. Cl.: C07C 211/63, C07C 59/125, C10M 129/26, C07C 209/68, C07C 51/41, C10M 129/40

(54) **IONIC LIQUIDS**
IONISCHE FLÜSSIGKEITEN
LIQUIDES IONIQUES

(30) Priority: 30.01.2014 EP 14153215
(43) Date of publication of application: 07.12.2016
(73) Proprietor: KAO CHEMICALS GmbH, 46446 Emmerich (DE)
(72) Inventor: BENDER, Holger, 46446 Emmerich (DE); HOUDKAMP, Rob, 46446 Emmerich (DE); DENZER, Horst, 46446 Emmerich (DE); MYRDEK, Thomas, 46446 Emmerich (DE); VAN DER VEEN, Reinout, 46446 Emmerich (DE)
(74) Representative: Hoffmann Eitle
(86) International application number: PCT/EP2015/051347
(87) International publication number: WO 2015/113906

(56) References cited:
- US-A1- 2010 137 175
- H. KONDO: "Protic ionic liquids with ammonium salts as lubricants for magnetic thin film media", TRIBOLOGY LETTERS, vol. 31, 2008, pages 211-218, XP002726119,

## Description

### FIELD OF THE INVENTION

The present invention relates to novel ionic liquids comprising polyethercarboxylate groups as anions, alkyl ammonium groups as cations, a process of preparation of said ionic liquids and its use in high duty lubricant.

### STATE OF THE ART

Ionics liquids are generally understood to be organic salts, or mixtures of organic salts, having melting points below 100°C and being non-volatile, this is, with no detectable vapour pressure below the temperature of their thermal decomposition.
The fact that ionic liquids are only comprised of cations and anions explains their distinctive properties, furthermore, the choice of ions will determine physical and chemical properties such as thermal stability, melting point, low vapour pressure, solubility, electric conductivity, solvent properties, electroelasticity, high heat capacity and non-flammability.

Because of their particular properties, ionic liquids are attracting attention in many fields, finding applications in electroelastic materials, analytics, solvents, liquids crystals, heat storage or electrolytes.

One of the applications of ionic liquids is in lubricants as being advantageous regarding several performance requirements.

Relevant properties for high performance lubricants are low viscosity, high thermal stability, low hygroscopicity, low evaporation loss and large liquid range between glass transition temperature and decomposition temperature. High thermal stability extends the lifetime of lubricants and reduces debris particle formation due to thermal decomposition. Low hygroscopicity reduces corrosion problems. Low viscosity is necessary to obtain good flowability and to avoid tribocorrosion in lubricants. Large liquid ranges and low evaporation loss permit the use of lubricants under high temperature conditions.

The most common ionic liquids comprise anionic components such as halostannates, haloaluminates, hexafluorophosphates or tetrafluoroborates combined with substituted ammonium, phosphonium, pyridinium or imidazolium cations. The state of the art discloses the occurrence of relevant performance features in some specific combinations.
For instance, US2010/0204074 describes ionic liquids, liquid at room temperature, wherein the cation can be chosen among tetra-alkyl ammonium salts, benzyl trialkyl ammonium salts, tetra-alkyl phosphonium salts and benzyl trialkyl phosphonium salts, wherein at least 2 alkyl groups are 8-10 carbon residues, and not all the carbon residues are identical. The ionic liquid is used as a solvent, extractant, phase transfer catalyst and as heat-exchange medium additive.
An especially relevant piece of prior art relevant to the present invention is US2010/0137175. The use of ionic liquid comprising ethercarboxylate anion compounds is described, wherein the cations can be selected among alkali or alkaline earth metal ions, ammonium, oxonium, sulphonium or phosphonium groups, and the anions correspond to an ethercarboxylate group with a hydrocarbon group which has from 1 to 3 carbon atoms. The ionic liquids are mainly used as solvents and as extractants.

The present invention provides a novel ionic liquid comprising polyethercarboxylates compounds as anions and alkyl ammonium compounds as cations, a process of preparation of said ionic liquids and its use in high duty lubricants, wherein the ionic liquids of the invention are liquid at room temperature, show excellent values of hygroscopicity, as well as high thermal stabilities and reduced values of viscosity.

### SUMMARY OF THE INVENTION

The first object of the present invention is an ionic liquid comprising ethercarboxylate compounds as anions and alkyl ammonium compounds as cations.
Another object of the present invention is the process to obtain said ionic liquids.
The third object of the present invention is the use of said ionic liquids in high duty lubricants.

### DETAILED DESCRIPTION OF THE INVENTION

### Ionic liquid

The main object of the present invention is an ionic liquid comprising at least a compound (a)
wherein,
compound (a) comprises one or more products of formula (I)

**X⁺ Y⁻** **Formula (I)**

wherein
the anion Y⁻ is at least one ether carboxylate anion of formula (II), or a mixture thereof 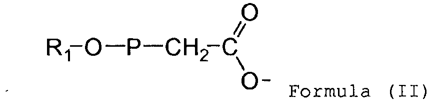 wherein P consists of
n units of -CH₂CH₂O- and m units of -CH₂CHRO- and/or -CHRCH₂O-, wherein n represents a number from 2 to 8, m represents a number from 0 to 6, and the sum of n + m represents the average alkoxylation degree which corresponds to a number from 2 to 14; and
wherein
R represents an alkyl group containing 1 to 2 carbon atoms;
R₁ represents a linear or branched alkyl having 6 to 22 carbon atoms or a linear alkenyl group having 6 to 22 carbon atoms;
and the cation X⁺ is an alkyl ammonium cation of formula (III), or a mixture thereof 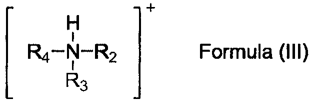 wherein
   R₂ represents a linear or branched alkyl, a linear alkenyl or a linear alkynyl group having 6 to 24 carbon atoms;
   R₃ represents a linear or branched alkyl group having 1 to 5 carbon atoms; R₄ represents a linear or branched alkyl group having 1 to 5 carbon atoms, or a linear or branched alkyl, a linear alkenyl or a linear alkynyl group having 6 to 24 carbon atoms.

In one embodiment of the present invention, the anion Y⁻ consists of one or more ethercarboxylate anion compounds of formula (II), wherein R₁ represents a linear or branched alkyl having 6 to 22 carbon atoms or a linear alkenyl group having 6 to 22 carbon atoms; preferably the alkyl or alkenyl group contains from 8 to 18 carbon atoms, more preferably from 12 to 18 carbon atoms; n (-CH₂CH₂O- units) represents a number within the range from 2 to 8, more preferably from 2 to 6, and m (-CH₂CHRO- and/or -CHRCH₂O-units) represents a number within the range from 0 to 6, preferably from 0 to 3, more preferably 0, and the sum of n + m represents the average alkoxylation degree which corresponds to a number from 2 to 14, preferably from 2 to 9, more preferably from 2 to 6.

In one embodiment of the present invention, the anion Y⁻ consists of one or more ethercarboxylate anion compounds of formula (II), wherein R₁ represents a linear or branched alkyl having 6 to 22 carbon atoms or a linear alkenyl group having 6 to 22 carbon atoms and up to 3 double bonds; preferably the alkyl or alkenyl group contains from 8 to 18 carbon atoms, more preferably from 12 to 18 carbon atoms; n (-CH₂CH₂O- units) represents a number from 2 to 8, more preferably from 2 to 6, and m (-CH₂CHRO- and/or - CHRCH₂O- units) represents a number from 0 to 6, preferably from 0 to 3, more preferably 0, and the sum of n + m represents the average alkoxylation degree which corresponds to a number from 2 to 14, preferably from 2 to 9, more preferably from 2 to 6.

The order or sequence of the -(CH₂CH₂O)- , -(CH₂CHRO)- ,or -(CHRCH₂O)-groups, wherein R represents an alkyl group containing 1 to 2 carbon atoms, is not critical for the invention. Thus, both ether carboxylate anions of the present invention comprising -(CH₂CH₂O)- and -(CH₂CHRO)- or - (CHRCH₂O) - groups in separated blocks and ether carboxylate anions of the present invention comprising randomly distributed -(CH₂CH₂O)- and -(CH₂CHRO)- or - (CHRCH₂O)- groups may be used according to the invention.

In another embodiment of the present invention, the cation X⁺ consists of one or more alkyl ammonium cation compounds of formula (III), wherein R₂ represents a linear or branched alkyl, a linear alkenyl or a linear alkynyl group having 6 to 24 carbon atoms, preferably 10 to 18 carbon atoms, R₃ represents a linear or branched alkyl group having 1 to 5 carbon atoms, preferably a methyl group, R₄ represents a linear or branched alkyl group having 1 to 5 carbon atoms, preferably a methyl group, or a linear or branched alkyl, a linear alkenyl or a linear alkynyl group having 6 to 24 carbon atoms, preferably from 10 to 18 carbon atoms.

In another embodiment of the present invention, the ionic liquid of formula (I) preferably comprises an ether carboxylate anion of formula (II) wherein R₁ represents a linear or branched alkyl or a linear alkenyl group having 6 to 22 carbon atoms, preferably 8 to 18 carbon atoms, more preferably having 12 to 18 carbon atoms, the n in the -CH₂CH₂O- units represents a number from 2 to 8, more preferably from 2 to 6, and the m in the -CH₂CHRO- and/or - CHRCH₂O- units represents a number from 0 to 6, preferably from 0 to 3, more preferably 0, and the sum of n + m represents the average alkoxylation degree which corresponds to a number from 2 to 14, preferably from 2 to 9, more preferably from 2 to 6, and comprises an ammonium cation of formula (III) wherein R₂ represents a linear or branched alkyl, a linear alkenyl or a linear alkynyl group having 6 to 24 carbon atoms, preferably 10 to 18 carbon atoms, R₃ represents a linear or branched alkyl group having 1 to 5 carbon atoms, preferably a methyl group, R₄ represents a linear or branched alkyl group having 1 to 5 carbon atoms, preferably a methyl group.

In another embodiment of the present invention, the ionic liquid of formula (I) preferably comprises an ether carboxylate anion of formula (II) wherein R₁ represents a linear or branched alkyl or a linear alkenyl group having 6 to 22 carbon atoms, preferably 8 to 18 carbon atoms, more preferably having 12 to 18 carbon atoms, wherein the alkenyl has up to 3 double bonds, the n in the -CH₂CH₂O- units represents a number from 2 to 8, more preferably from 2 to 6, and the m in the -CH₂CHRO- and/or -CHRCH₂O- units represents a number from 0 to 6, preferably from 0 to 3, more preferably 0, and the sum of n + m represents the average alkoxylation degree which corresponds to a number from 2 to 14, preferably from 2 to 9, more preferably from 2 to 6, and comprises an ammonium cation of formula (III) wherein R₂ represents a linear or branched alkyl, a linear alkenyl or a linear alkynyl group having 6 to 24 carbon atoms, preferably 10 to 18 carbon atoms, R₃ represents a linear or branched alkyl group having 1 to 5 carbon atoms, preferably a methyl group, R₄ represents a linear or branched alkyl group having 1 to 5 carbon atoms, preferably a methyl group.

In another embodiment of the present invention, the ionic liquid of formula (I) preferably comprises an ether carboxylate anion of formula (II) wherein R₁ represents a linear or branched alkyl or a linear alkenyl group having 6 to 22 carbon atoms, preferably having 8 to 18 carbon atoms, more preferably having 12 to 18 carbon atoms, the n in the -CH₂CH₂O- units represents a number within the range from 2 to 8, more preferably from 2 to 6, and the m in the -CH₂CHRO- and/or -CHRCH₂O- units represents a number within the range from 0 to 6, preferably from 0 to 3, more preferably 0, and the sum of n + m represents the average alkoxylation degree which corresponds to a number from 2 to 14, preferably from 2 to 9, more preferably from 2 to 6 and comprises an ammonium cation of formula (III) wherein R₂ represents a linear or branched alkyl, a linear alkenyl or a linear alkynyl group having 6 to 24 carbon atoms, preferably 10 to 18 carbon atoms, R₃ represents a linear or branched alkyl group having 1 to 5 carbon atoms, preferably a methyl group, R₄ represents a linear or branched alkyl, a linear alkenyl or a linear alkynyl group having 6 to 24 carbon atoms, preferably 10 to 18 carbon atoms.

In another embodiment of the present invention, the ionic liquid of formula (I) preferably comprises an ether carboxylate anion of formula (II) wherein R₁ represents a linear or branched alkyl or a linear alkenyl group having 6 to 22 carbon atoms, preferably having 8 to 18 carbon atoms, more preferably having 12 to 18 carbon atoms, wherein the alkenyl has up to 3 double bonds, the n (-CH₂CH₂O- units) represents a number from 2 to 8, more preferably from 2 to 6, and the m (-CH₂CHRO- and/or -CHRCH₂O- units) represents a number from 0 to 6, preferably from 0 to 3, more preferably 0, and the sum of n + m represents the average alkoxylation degree which corresponds to a number from 2 to 14, preferably from 2 to 9, more preferably from 2 to 6, and comprises an ammonium cation of formula (III) wherein R₂ represents a linear or branched alkyl, a linear alkenyl or a linear alkynyl group having 6 to 24 carbon atoms, preferably 10 to 18 carbon atoms, R₃ represents a linear or branched alkyl group having 1 to 5 carbon atoms, preferably a methyl group, R₄ represents a linear or branched alkyl, a linear alkenyl or a linear alkynyl group having 6 to 24 carbon atoms, preferably 10 to 18 carbon atoms.

In one embodiment, the anion Y⁻ consists of at least one selected from Laureth-3 carboxylate, Laureth-4 carboxylate, Laureth-5 carboxylate, Laureth-6 carboxylate and Capryleth-6 carboxylate.

In one embodiment, the cation X⁺ consists of at least one selected from dodecyldimethyl ammonium and didodecylmethyl ammonium.

In one embodiment, the anion Y⁻ consists of at least one selected from Laureth-3 carboxylate, Laureth-4 carboxylate, Laureth-5 carboxylate, Laureth-6 carboxylate, Capryleth-6 carboxylate and the cation X⁺ consists of at least one selected from dodecyldimethyl ammonium and didodecylmethyl ammonium.

In another embodiment of the invention, the ionic liquid according to the invention further comprises at least a compound (b). Compound (b) represents one or more impurities which may be present in the ionic liquid. Typical impurities (compound (b)) are unreacted substances in the process for obtaining the ether carboxylic acid as precursor of the ether carboxylate anion compound, such as not carboxymethylated fatty alcohol polyglycol ether, or by-products from the same reaction like sodium glycolate, sodium diglycolate, substances having a low vapour pressure, high boiling substances, oligomer and polymer as well as traces of inorganic halides like sodium chloride and traces of water.

The presence of impurities can influence the properties of ionic liquids; therefore, the performance of the ionic liquid can also be affected by the impurities, which are in case of ionic liquids all non-ionic organic compounds, inorganic salts and water.
The ionic liquid of the present invention comprises at least a compound (a) and optionally comprises at least a compound (b). Compound (b) represents one or more of the above mentioned impurities and can be present in the ionic liquid in an amount of less than 5% by weight based on the total amount of the ionic liquid.

### Preparation process

The ionic liquid of the present invention can be prepared by a one-pot process reaction by reacting an ether carboxylic acid as precursor for the ether carboxylate anion of formula (II) and an amine as precursor for the alkyl ammonium cation of formula (III) in the presence of a solvent, at 30°C. The second step of the process consists in the evaporation of the solvent under reduced pressure and drying under high vacuum conditions at 40°C for 24h.

How to obtain an ether carboxylic acid as the precursor for the ether carboxylate anion component of the ionic liquid according to the invention is known by the person skilled in the art. Said ether carboxylic acid can be obtained by a Williamson reaction, this is, by the reaction of the corresponding alkylpolyoxyethylene alcohol with a halocarboxylic acid. In order to obtain a purified ether carboxylic acid, the reaction can be carried out by the reaction of the alkylpolyoxyethylene alcohol with metallic sodium. The isolation and purification process is already known by the person skilled in the art, and can be performed, for example, by means of vacuum distillation.

Suitable examples of ether carboxylic acids that can be used as precursors for the ether carboxylate anion of formula (II) according to the invention are commercially available ether carboxylic acids like AKYPO® RLM 45 CA (Laureth-6 carboxylic acid), AKYPO® LF 1 (Capryleth-6 carboxylic acid), AKYPO® RLM 25 (Laureth-4 carboxylic acid), AKYPO® RO 20 VG. (Oleth-3 carboxylic acid), AKYPO® RO 50 VG (Oleth-6 carboxylic acid), AKYPO® LF 2 (Capryleth-9 carboxylic acid), AKYPO® LF 4 (Mixture of Capryleth-9 carboxylic acid and Hexeth-4 carboxylic acid), all of them marketed by KAO Chemicals Europe. A purification step of the ether carboxylic acid can be carried out if necessary due to NaCl content.
How to obtain the alkylamine as precursor for the alkyl ammonium cation is known by the person skilled in the art. It can be obtained by reacting fatty alcohols with NH₃, with alkylamines (RNH₂) or with dialkylamines (RR'NH), the suitable pressure, temperature conditions and catalysts being well known, as described in the patent applications EP-A-0500038 or EP-A-0908444.

Suitable amine as precursor for the ammonium cation of formula (III) according to the invention are dimethyloctylamine, dimethyldecylamine, dimethyllaurylamine, dimethylmyristylamine, dimethylpalmitylamine, dimethylcetylamine, dimethylstearylamine, dimethylbehenylamine, coconut dimethylalkylamine, oleic dimethylalkylamine, tallow dimethylalkylamine, totally or partially hydrogenated tallow dimethyl-alkylamine, or mixtures thereof. Other suitable amines are methyldioctylamine, methyldidecylamine, methyldilaurylamine, methyldimyristylamine, methyldipalmitylamine, methyldicetylamine, methyldistearylamine, methyldibehenylamine, coconut methyldialkylamine, oleic methyldialkylamine, tallow methyldialkylamine, totally or partially hydrogenated tallow methyldialkylamine, or mixtures thereof.

Examples of commercially available amines are those corresponding to the commercial reference FARMIN® DM2098 (N,N-Dimethyldodecylamine), FARMIN® DM4098 (N,N-Dimethyltetradecylamine), FARMIN® DM6098 (N,N-Dimethylhexadecylamine), FARMIN® DM8098 (Octadecyldimethylamine), FARMIN® DM2471 (N,N-Dimethylalkylamine), FARMIN® DM-O (oleic dimethylalkylamine), FARMIN® M2-2095 (didodecylmonomethylamine), FARMIN® DM-T (tallow dimethylalkylamine, FARMIN® DM-TH (hydrogenated tallow dimethylalkylamine), FARMIN® M-D10 (methyldidecylamine), FARMIN® M-2TH (hydrogenated tallow methyldialkylamine), all of them marketed by KAO Chemicals Europe.

### Use of ionic liquids in high duty lubricants

The ionic liquids of the present invention can be used in high-duty lubricants.
The use of the ionic liquids of the present invention in high-duty lubricants can be explained due to their good values of thermal stability, viscosity, hygroscopicity and liquid range (all ionic liquids of the present invention are liquid at room temperature).

Viscosity is one of the most important parameters of lubricant oils. Low viscosity means good flowability but a lubricant also needs to provide high load bearing capacity to enable hydrodynamic lubrication conditions. Due to their ionic nature, ionic liquids form a more stable lubricant film with higher load bearing capacity on metal surfaces than neutral paraffinic lube oils.
Thermal stability is another key parameter for high duty lubricants: most petroleum based lubricants decompose at temperatures above 150°C, whereas the ionic liquids of the invention have decomposition temperatures significantly higher than 150°C.
The limitation of use in a lubricant of a substance due to the water content is known. Low hygroscopicity values of the ionic liquids of the present invention reduce corrosion problems associated to lubricants.

The following examples are given in order to provide a person skilled in the art with a sufficiently clear and complete explanation of the present invention, but should not be considered as limiting the essential aspects of its subject, as set out in the preceding portions of this description.

### EXAMPLES

### Example 1: Synthesis of Dodecyldimethylammonium Laureth-5 carboxylate:

### Synthesis of laureth-5 carboxylic acid:

First, dodecanetetraoxyethylene alcohol was prepared. 0.2 eq NaOH-50%wt was dissolved in tetraethyleneglycol and heated while stirring to 100°C. Then 1-bromododecane (0.2 eq) was added dropwise within 20 minutes. After stirring overnight the reaction mixture was cooled down to room temperature and extracted 3 times with hexane.
The organic phase subsequently was washed with saturated sodium chloride solution and dried 2 hours over sodium sulphate. Hexane was removed by rotational evaporation, and the raw product was distilled under high vacuum (10-7 mbar) for purification.
The dodecanetetraoxyethylenealcohol was then dissolved in xylene and heated to 90°C. Metallic sodium (1 eq) was added in small portions within 30 minutes under nitrogen atmosphere. The mixture was stirred at 90°C for 4h. Dry, finely powdered sodium monochloroacetate(SMCA) (1eq) was added portionwise under nitrogen atmosphere. The reaction mixture was cooled to room temperature, 100 ml of water and 100 ml of diethylether were added. The mixture was acidified by dropwise addition of concentrated HCl to a pH<2. Phases were separated and the solvents (xylene and diethylether) were evaporated from the organic phase. The residue was dissolved in 100 ml of n-hexane and the product was crystallized in a refrigerator (4°C). Furthermore, recrystallization from n-hexane was repeated 5 times. Before the last crystallization, the organic phase was dried over sodium sulphate and then n-hexane was evaporated in vacuo.

### Synthesis of Dodecyldimethylamine:

600 g of dodecyl alcohol and 3 g (corresponding to 0.5% by weight based on the alcohol used as the raw material) of a copper-nickel catalyst were fed into a 1-L four-necked flask. The system was purged with nitrogen while stirring and the temperature rise of the system was initiated. When the temperature of the system reached 100°C hydrogen gas was blown into the system at a flow rate of 40 1/hr with a flowmeter and the temperature of the system was raised to a reaction temperature, of 200°C. At this temperature, the introduction of dimethylamine gas was initiated and a reaction was conducted for 5 hours. After the completion of the reaction, the reaction mixture was filtered to remove the catalyst, thereby providing crude N,N-dimethyldodecylamine. The crude N,N-dimethyldodecylamine was put in a 1-L four-necked flask, followed by the addition of 3 g of activated carbon and 3 g of Kyoward 600 S (a product of Kyowa Chemical Industry, main components: silica 64.9%, magnesia 13.5%). The contents were stirred at 90°C in a nitrogen atmosphere for about 2 hours and filtered to remove the adsorbent. The filtrate was purified by distillation (5 Torr) to obtain dodecyldimethylamine.

### Preparation of Ionic Liquid:

Equimolar amounts of laureth-5 carboxylic acid and ammonium base (dodecyldimethylamine) were dissolved in ethanol and stirred at room temperature for 30 min. Afterwards the solvent was evaporated under reduced pressure. The resulting ionic liquid was dried under high vacuum at 40°C for at least 24h.

### Example 2:

### Synthesis of laureth-3 carboxylic acid:

The procedure of Example 1 is repeated using diethyleneglycol and 1-bromododecane as starting materials of the synthesis.

### Synthesis of Dodecyldimethylamine

The dodecyldimethylamine has been prepared using the same procedure as in Example 1.

### Preparation of Ionic Liquid:

The same procedure as in Example 1 has been performed.

### Example 3:

### Synthesis of capryleth-6 carboxylic acid:

Capryleth-6 carboxylic acid was prepared by the modified Williamson synthesis, which is nowadays the industrial standard process for alkyl ether carboxylic acid synthesis.
370g of the octyl alcohol was ethoxylated with 630g ethylene oxide in the presence of 2 g NaOH-50%. In order to remove the most difficult to eliminate carboxymethylate by-products, a distillation under high vacuum was done. The 820 g of ethoxylate were treated with SMCA, 370 g in the presence of 130 g NaOH solid. For the separation of the water soluble by-products: water, HCl and some NaCl were added to the crude and the mixture was heated resulting in a good separation of the alkyl ether carboxylic acid (top layer) from the aqueous layer. For NaCl removal from the capryleth-6 carboxylic acid, these ether carboxylic acids were desalted by water removal under vacuum and removal of the precipitated NaCl by centrifugation followed by a decantation step.

### Synthesis of Didodecylmethylamine

600 g of dodecyl alcohol and 3 g (corresponding to 0.5% by weight based on the alcohol used as the raw material) of a copper-nickel catalyst were fed into a 1 l four-necked flask. The system was purged with nitrogen while stirring and the temperature of the system was initiated to heat. When the temperature of the system reached 100°C, hydrogen gas was blown into the system at a flow rate of 40 1/hr with a flowmeter and the temperature of the system was raised to 200°C. At this temperature, the introduction of methylamine gas was initiated and the reaction was conducted for 7 hours. After the completion of the reaction, the reaction resultant was filtered to remove the catalyst, thereby providing crude N-methyldidodecylamine. 200g of the crude N-methyldidodecylamine was put in a 500 ml Hertz-flask. Using the other flask, KOH was weighed to be 0.2%, 0.5%, or 1.0% as compared with the crude N-methyldidodecylamine and dissolved by 1 g of deionized water. The solution of NaOH was put into the above-mentioned Hertz-flask. After setting on apparatus for a distillation (capillary distillation), a mixture in the Hertz-flask was conducted by bubbling with nitrogen gas under an atmospheric pressure at a temperature of 200°C for 2 hours. And then the pressure in the Hertz-flask was reduced to 5 to 10 Torr, and the mixture in the Hertz-flask was heated at 250°C. Further the mixture was distilled and separated at said temperature for 5 hours to obtain N-methyldidodecylamine.

### Preparation of Ionic Liquid:

Same procedure as in Example 1.

### Example 4:

### Synthesis of laureth-4 carboxylic acid:

655g of the lauryl alcohol was ethoxylated with 340g ethylene oxide in the presence of 2 g NaOH-50%. In order to remove the most difficult to eliminate carboxymethylate by-products, a distillation under high vacuum was done. The 830 g of ethoxylate were treated with SMCA, 460 g in the presence of 170 g NaOH solid. For separation of the water soluble by-products: water, HCl and some NaCl were added to the crude and the mixture was heated resulting in a good separation of the alkyl ether carboxylic acid (top layer) from the aqueous layer. For NaCl removal from the laureth-4 carboxylic acid, these ether carboxylic acids were desalted by water removal under vacuum and removal of the precipitated NaCl by centrifugation followed by a decantation step.

### Synthesis of Didodecylmethylamine

Same procedure as in Example 3.

### Preparation of Ionic Liquid:

Same procedure as in Example 1.

### Example 5:

### Synthesis of laureth-6 carboxylic acid:

490g of the lauryl alcohol was ethoxylated with 510g ethylene oxide in the presence of 2 g NaOH-50%. The 810 g of ethoxylate were treated with SMCA, 340 g in the presence of 125 g NaOH solid. For separation of the water soluble by-products: water, HCl and some NaCl were added to the crude and the mixture was heated resulting in a good separation of the alkyl ether carboxylic acid (top layer) from the aqueous layer. For NaCl removal from the laureth-6 carboxylic acid, these ether carboxylic acids were desalted by water removal under vacuum and removal of the precipitated NaCl by centrifugation followed by a decantation step.

### Synthesis of Didodecylmethylamine

Same procedure as in Example 3.

### Preparation of Ionic Liquid:

Same procedure as in Example 1.

**Table 1 summarizes all the synthesized ionic liquids:**

| **Example** | **Ionic Liquid** | **Ethercarboxylate anion (II) precursor** | **Ammonium cation (III) precursor** |
|---|---|---|---|
| 1 | Dodecyldimethylammonium laureth-5 carboxylate | Laureth-5 carboxylic acid | Dodecyldimethylamine |
| 2 | Dodecyldimethylammonium laureth-3 carboxylate | Laureth-3 carboxylic acid | Dodecyldimethylamine |
| 3 | Didodecylmethylammonium capryleth-6 carboxylate | Capryleth-6 carboxylic acid | Didodecyldimethylamine |
| 4 | Didodecylmethylammonium laureth-4 carboxylate | Laureth-4 carboxylic acid | Didodecyldimethylamine |
| 5 | Didodecylmethylammonium laureth-6 carboxylate | Laureth-6 carboxylic acid | Didodecyldimethylamine |

| | | | |
|---|---|---|---|
| Relevant properties for high duty lubricants: liquid range, thermal stability, viscosity, hygroscopicity and evaporation loss were determined for the ionic liquids prepared in Examples 1 to 5. | | | |

Table 2 summarizes the results obtained for the ionic liquids of the examples 1 to 5:

**Table 2**

| **Lubricant type** | **Liquid range** | **Thermal stability** | **Viscosity (mPas)** | **Hygroscopicity** | **Evaporation loss** |
|---|---|---|---|---|---|
| Dodecyldimethylammonium laureth-5 carboxylate | Tg:-41.8°C | Tdec:250°C | 20°C:100 | 1.6% water after 48h | 0% wt loss |
| | Tdec:250°C | | 40°C:40 | | |
| | ΔT:291.8°C | | 80°C:10 | | |
| Dodecyldimethylammonium laureth-3 carboxylate | Tg:-37.5°C | Tdec:230°C | 20°C:100 | 5.1% water after 48h | 0% wt loss |
| | Tdec:230°C | | 40°C:40 | | |
| | ΔT:267.5°C | | 80°C:10 | | |
| Didodecylmethylammonium capryleth-6 carboxylate | Tdec:285°C | Tdec:285°C | 20°C:80 | 5.5% water after 48h | 0% wt loss |
| | | | 80°C:10 | | |
| Didodecylmethylammonium laureth-4 carboxylate | Tdec:295°C | Tdec:295°C | 20°C: 110 | 1.6% water after 48h | 0% wt loss |
| | | | 80°C:20 | | |
| Didodecylmethylammonium laureth-6 carboxylate | Tdec:295°C | Tdec:295°C | 20°C: 110 | 1.6% water after 48h | 0% wt loss |
| | | | 80°C:20 | | |

Glass transition temperature (Tg) is measured according to ISO-11357-2 by Differential Scanning Calorimetry (DSC).
Decomposition temperature (Tdec) is measured according to ISO-11358 by Thermogravimetric analysis (TGA).
Viscosity is measured according to ISO 3210 / DIN 53018 by use of rotational rheometer with cone/plate system.
Hygroscopicity is measured by gravimetric method in % water absorption at 20°C within 48h.
Evaporation loss is measured according to ASTM D 5800, during 1h at 250°C.

Results of the lubricating performance of the ionic liquids of examples 1 and 5 are described in Table 3.

**Table 3**

| Lubricant type | Friction reduction during threading (N·cm) |
|---|---|
| Dodecyldimethylammonium laureth-5 carboxylate | 105 |
| Didodecylmethylammonium laureth - 6 carboxylate | 104 |

Lubricating performance is measured according to Tapping Torque test: ASTM D5619 (2011). Test is performed by mimicking a thread cutting process, the torque is measured as a function of the respective thread depth in a test bar (made of aluminium 3.4365). Results are expressed by mean torque (arithmetic average of the instantaneous tapping torque values through the depth of the hole).
Results shown in the reduction of friction during threading of the ionic liquids are better than results shown in conventional lubricants such as mineral refined oil and TMP trioleate.

From these results it can be seen that the ionic liquids of the present invention meet the necessary requirements for a good performance in a high duty lubricant.

## Claims

1. An ionic liquid comprising:
- at least a compound (a)
wherein
Compound (a) comprises one or more products of formula (I)
X⁺Y⁻ Formula (I)
wherein
the anion Y⁻ is an ether carboxylate anion or a mixture thereof of formula (II) 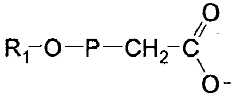 wherein P consists of
n units of -CH₂CH₂O- and m units of -CH₂CHRO- or -CHRCH₂O- wherein n represents a number from 2 to 8, m represents a number from 0 to 6,
and the sum of n+m represents the average alkoxylation degree which corresponds to a number from 2 to 14;and
wherein
R represents an alkyl group having 1 to 2 carbon atoms;
R₁ represents a linear or branched alkyl having from 6 to 22 carbon atoms or a linear alkenyl group having 6 to 22 carbon atoms;
and the cation X⁺ is an alkyl ammonium cation or a mixture thereof of formula (III) 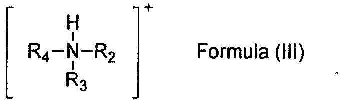 wherein
R₂ represents a linear or branched alkyl, a linear alkenyl or a linear alkynyl group having 6 to 24 carbon atoms,
R₃ represents a linear or branched alkyl group having 1 to 5 carbon atoms,
R₄ represents a linear or branched alkyl group having 1 to 5 carbon atoms, or a linear or branched alkyl, a linear alkenyl or a linear alkynyl group having 6 to 24 carbon atoms.

2. The ionic liquid according to claim 1, **characterized in that** the anion Y⁻ is an ether carboxylate anion of formula (II), wherein the alkenyl has up to 3 double bonds.

3. The ionic liquid according to any one of claims 1-2 wherein R₁ has 8 to 18 carbon atoms.

4. The ionic liquid according to any one of claims 1-3 wherein P consists of
n units of -CH₂CH₂O- and m units of -CH₂CHRO- or -CHRCH₂O-, wherein n represents a number from 2 to 6, m represents a number from 0 to 3, and the sum of n+m represents the average alkoxylation degree which corresponds to a number from 2 to 9.

5. An ionic liquid according to any one of claims 1 to 4, **characterized in that** the cation X⁺ is an alkyl ammonium cation of formula (III), wherein
R₂ represents a linear or branched alkyl, a linear alkenyl or a linear alkynyl group having 10 to 18 carbon atoms,
R₃ represents a methyl group,
R₄ represents a methyl group.

6. The ionic liquid according to any one of claims 1 to 4, **characterized in that** the cation X⁺ is an alkyl ammonium cation of formula (III), wherein
R₂ represents a linear or branched alkyl, a linear alkenyl or a linear alkynyl group having 10 to 18 carbon atoms,
R₃ represents a linear or branched alkyl group having 1 to 5 carbon atoms, preferably a methyl group,
R₄ represents a linear or branched alkyl, a linear alkenyl or a linear alkynyl group having 10 to 18 carbon atoms.

7. The ionic liquid according to claim 1 wherein the anion Y⁻ is at least one selected from Laureth-3 carboxylate, Laureth-4 carboxylate, Laureth-5 carboxylate, Laureth-6 carboxylate and Capryleth-6 carboxylate.

8. The ionic liquid according to any one of claims 1 to 7 wherein the cation X⁺ is at least one selected from dodecyldimethyl ammonium and didodecylmethyl ammonium.

9. A process for preparing the ionic liquid of formula (I) according to any one of claims 1 to 8, wherein the ionic liquid of formula (I) is prepared by a one-pot process reaction by reacting an ether carboxylic acid as precursor for the ether carboxylate anion of formula (II) and an alkyl amine as precursor for the alkyl ammonium cation of formula (III) in the presence of a solvent, followed by the evaporation of the solvent under reduced pressure and drying under high vacuum conditions.

10. Use of an ionic liquid according to any one of claim 1 to 8 in a lubricant.

## Patentansprüche

1. Ionische Flüssigkeit, umfassend:
- mindestens eine Verbindung (a)
wobei
Verbindung (a) ein oder mehrere Produkte der Formel (I) umfasst
X⁺ Y⁻ Formel (I)
worin
das Anion Y⁻ ein Ethercarboxylatanion mit der Formel (II) oder eine Mischung davon ist 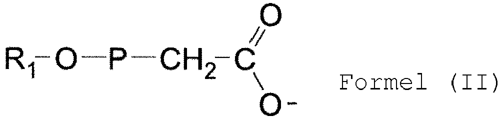 worin P aus
n Einheiten -CH₂CH₂O- und m Einheiten -CH₂CHRO- oder -CHRCH₂O-besteht, wobei n eine Zahl von 2 bis 8 bedeutet, m eine Zahl von 0 bis 6 bedeutet und die Summe von n+m den mittleren Alkoxylierungsgrad bedeutet, der einer Zahl von 2 bis 14 entspricht; und
worin
R eine Alkylgruppe mit 1 bis 2 Kohlenstoffatomen bedeutet;
R₁ eine lineare oder verzweigte Alkylgruppe mit 6 bis 22 Kohlenstoffatomen oder eine lineare Alkenylgruppe mit 6 bis 22 Kohlenstoffatomen bedeutet;
und das Kation X⁺ ein Alkylammoniumkation mit der Formel (III) oder eine Mischung davon ist 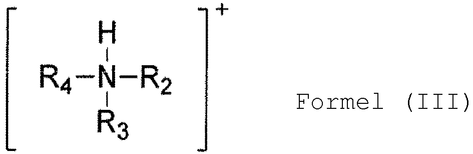 worin
R₂ eine lineare oder verzweigte Alkylgruppe, eine lineare Alkenylgruppe oder eine lineare Alkinylgruppe mit 6 bis 24 Kohlenstoffatomen bedeutet,
R₃ eine lineare oder verzweigte Alkylgruppe mit 1 bis 5 Kohlenstoffatomen bedeutet,
R₄ eine lineare oder verzweigte Alkylgruppe mit 1 bis 5 Kohlenstoffatomen, oder eine lineare oder verzweigte Alkylgruppe, eine lineare Alkenylgruppe oder eine lineare Alkinylgruppe mit 6 bis 24 Kohlenstoffatomen bedeutet.

2. Ionische Flüssigkeit gemäß Anspruch 1, **dadurch gekennzeichnet, dass** das Anion Y⁻ ein Ethercarboxylatanion der Formel (II) ist, worin die Alkenylgruppe bis zu 3 Doppelbindungen aufweist.

3. Ionische Flüssigkeit gemäß mindestens einem der Ansprüche 1-2, wobei R₁ 8 bis 18 Kohlenstoffatome aufweist.

4. Ionische Flüssigkeit gemäß mindestens einem der Ansprüche 1-3, wobei P aus
n Einheiten -CH₂CH₂O- und m Einheiten -CH₂CHRO- oder -CHRCH₂O-besteht, wobei n eine Zahl von 2 bis 6 bedeutet, m eine Zahl von 0 bis 3 bedeutet und die Summe von n+m den mittleren Alkoxylierungsgrad bedeutet, der einer Zahl von 2 bis 9 entspricht.

5. Ionische Flüssigkeit gemäß mindestens einem der Ansprüche 1 bis 4, **dadurch gekennzeichnet, dass** das Kation X⁺ ein Alkylammoniumkation der Formel (III) ist, worin
R₂ eine lineare oder verzweigte Alkylgruppe, eine lineare Alkenylgruppe oder eine lineare Alkinylgruppe mit 10 bis 18 Kohlenstoffatomen bedeutet,
R₃ eine Methylgruppe bedeutet,
R₄ eine Methylgruppe bedeutet.

6. Ionische Flüssigkeit gemäß mindestens einem der Ansprüche 1 bis 4, **dadurch gekennzeichnet, dass** das Kation X⁺ ein Alkylammoniumkation der Formel (III) ist, worin
R₂ eine lineare oder verzweigte Alkylgruppe, eine lineare Alkenylgruppe oder eine lineare Alkinylgruppe mit 10 bis 18 Kohlenstoffatomen bedeutet,
R₃ eine lineare oder verzweigte Alkylgruppe mit 1 bis 5 Kohlenstoffatomen, vorzugsweise eine Methylgruppe, bedeutet,
R₄ eine lineare oder verzweigte Alkylgruppe, eine lineare Alkenylgruppe oder eine lineare Alkinylgruppe mit 10 bis 18 Kohlenstoffatomen bedeutet.

7. Ionische Flüssigkeit gemäß Anspruch 1, wobei das Anion Y⁻ mindestens ein Vertreter, ausgewählt aus Laureth-3 Carboxylat, Laureth-4 Carboxylat, Laureth-5 Carboxylat, Laureth-6 Carboxylat und Capryleth-6 Carboxylat, ist.

8. Ionische Flüssigkeit gemäß mindestens einem der Ansprüche 1 bis 7, wobei das Kation X⁺ mindestens ein Vertreter, ausgewählt aus Dodecyldimethylammonium und Didodecylmethylammonium, ist.

9. Verfahren zur Herstellung der ionischen Flüssigkeit der Formel (I) gemäß mindestens einem der Ansprüche 1 bis 8, worin die ionische Flüssigkeit der Formel (I) in einer Eintopfverfahrensreaktion durch Reagieren einer Ethercarbonsäure als Vorläufer für das Ethercarboxylatanion der Formel (II) und eines Alkylamins als Vorläufer für das Alkylammoniumkation der Formel (III) in Gegenwart von Lösungsmittel, gefolgt von Verdampfen des Lösungsmittels bei vermindertem Druck und Trocknen unter Hochvakuumsbedingungen, hergestellt wird.

10. Verwendung einer ionischen Flüssigkeit gemäß mindestens einem der Ansprüche 1 bis 8 in einem Schmiermittel.

## Revendications

1. Liquide ionique comprenant:
- au moins un composé (a)
dans lequel
le composé (a) comprend un ou plusieurs produits de formule (I)
X⁺Y⁻ Formule (I)
dans laquelle
l'anion Y⁻ est un anion carboxylate d'éther ou un mélange de ceux-ci de formule (II) 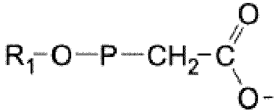 dans laquelle P est constitué de
n motifs -CH₂CH₂O- et m motifs -CH₂CHRO- ou -CHRCH₂O- dans lequel n représente un nombre de 2 à 8, m représente un nombre de 0 à 6, et la somme n + m représente le degré moyen d'alcoxylation qui correspond à un nombre de 2 à 14 ; et
dans laquelle
R représente un groupe alkyle ayant 1 à 2 atomes de carbone ;
R₁ représente un alkyle linéaire ou ramifié ayant 6 à 22 atomes de carbone ou un groupe alcényle linéaire ayant 6 à 22 atomes de carbone ;
et le cation X⁺ est un cation alkyl ammonium ou un mélange de ceux-ci de formule (III) 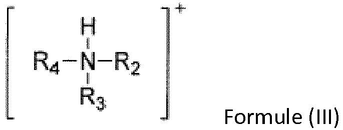 dans laquelle
R₂ représente un groupe alkyle linéaire ou ramifié, un groupe alcényle linéaire ou un groupe alcényle linéaire ayant 6 à 24 atomes de carbone,
R₃ représente un groupe alkyle linéaire ou ramifié ayant 1 à 5 atomes de carbone,
R₄ représente un groupe alkyle linéaire ou ramifié ayant 1 à 5 atomes de carbone, ou un groupe alkyle linéaire ou ramifié, un groupe alcényle linéaire ou un groupe alcynyle linéaire ayant 6 à 24 atomes de carbone.

2. Liquide ionique selon la revendication 1, **caractérisé en ce que** l'anion Y⁻ est un anion carboxylate d'éther de formule (II), dans lequel l'alcényle a jusqu'à 3 doubles liaisons.

3. Liquide ionique selon l'une quelconque des revendications 1 et 2 dans lequel R₁ a 8 à 18 atomes de carbone.

4. Liquide ionique selon l'une quelconque des revendications 1 à 3 dans lequel P est constitué de
n motifs -CH₂CH₂O- et m motifs -CH₂CHRO- ou -CHRCH₂O-, dans lequel n représente un nombre de 2 à 6, m représente un nombre de 0 à 3, et la somme n + m représente le degré moyen d'alcoxylation qui correspond à un nombre de 2 à 9.

5. Liquide ionique selon l'une quelconque des revendications 1 à 4, **caractérisé en ce que** le cation X⁺ est un cation alkyl ammonium de formule (III), dans lequel
R₂ représente un groupe alkyle linéaire ou ramifié, un groupe alcényle linéaire ou un groupe alcynyle linéaire ayant 10 à 18 atomes de carbone,
R₃ représente un groupe méthyle,
R₄ représente un groupe méthyle.

6. Liquide ionique selon l'une quelconque des revendications 1 à 4, **caractérisé en ce que** le cation X⁺ est un cation alkyl ammonium de formule (III), dans lequel
R₂ représente un groupe alkyle linéaire ou ramifié, un groupe alcényle linéaire ou un groupe alcynyle linéaire ayant 10 à 18 atomes de carbone,
R₃ représente un groupe alkyle linéaire ou ramifié ayant 1 à 5 atomes de carbone, de préférence un groupe méthyle,
R₄ représente un groupe alkyle linéaire ou ramifié, un groupe alcényle linéaire ou un groupe alcynyle linéaire ayant 10 à 18 atomes de carbone.

7. Liquide ionique selon la revendication 1 dans lequel l'anion Y⁻ est au moins l'un sélectionné parmi le carboxylate de laureth-3, le carboxylate de laureth-4, le carboxylate de laureth-5, le carboxylate de laureth-6 et le carboxylate de capryleth-6.

8. Liquide ionique selon l'une quelconque des revendications 1 à 7 dans lequel le cation X⁺ est au moins l'un sélectionné parmi le dodécyldiméthyl ammonium et le didodécylméthyl ammonium.

9. Procédé de préparation du liquide ionique de formule (I) selon l'une quelconque des revendications 1 à 8, dans lequel le liquide ionique de formule (I) est préparé par une réaction de procédé monotope par la réaction d'un éther d'acide carboxylique en tant que précurseur pour l'anion carboxylate d'éther de formule (II) et d'une alkyl aminé en tant que précurseur pour le cation alkyl ammonium de formule (III) en présence d'un solvant, suivie de l'évaporation du solvant sous pression réduite et d'un séchage dans des conditions de vide poussé.

10. Utilisation d'un liquide ionique selon l'une quelconque des revendications 1 à 8 dans un lubrifiant.
